# EUROPEAN PATENT APPLICATION

(11) **EP 1 094 072 A1**
(43) Date of publication of application: **25.04.2001**
(21) Application number: 99203402.5
(22) Date of filing: 18.10.1999
(51) Int. Cl.: C07K 14/575, C07K 7/64, A61K 38/35, A61K 38/12, A61P 5/08

(54) **Peptides having corticotropin releasing factor (CRF) antagonist activity**

(71) Applicant: DUPHAR INTERNATIONAL RESEARCH B.V, 1381 CP Weesp (NL)
(72) Inventor: The designation of the inventor has not yet been filed
(74) Representative: Muis, Maarten

(57) **Abstract**

The invention relates to a number of novel small peptides consisting of 12,15 or 16 amino acids.

The peptides are derived from Astressin, i.e. a peptide of 30 amino acids. It has surprisingly been found that these small peptides have also good CRF antagonistic activity.

## Description

The present invention relates to novel peptides, to a method for the preparation of these novel peptides, to pharmaceutical compositions containing one or more of these peptides as an active component and to methods of using these in the treatment of a wide range of stress-related disorders. The peptides have Corticotropin Releasing Factor (CRF) antagonist activity.

CRF, a 41 amino acid peptide, is the primary physiological regulator of proopiomelanocortin(POMC)-derived peptide secretion from the anterior pituitary gland (*Proc. Nat. Acad. Sci (USA) 80:4851 (1983); Science 213:1394 (1981))*. In addition to its endocrine role at the pituitary gland, immunohistochemical localization of CRF has demonstrated that the hormone has a broad extrahypothalamic distribution in the central nervous system. CRF is known to produce a wide spectrum of autonomic, electrophysiological and behavioural effects consistent with a neurotransmitter or neuromodulator role in brain. To date, two CRF receptor subtypes (CRF₁ and CRF₂), each with multiple splice variants, have been cloned ( *DeSouza, E.B. et al., Ann Rep. Med. Chem.(1995), 30, 21)*

Both preclinical and clinical data (relating to CRF levels, effects of exogenously administered CRF and CRF receptor density) support the hypothesis that CRF has an important role in a wide range of stress-related disorders (see: *Exp. & Clin. Endocrinology & Diabetes (1997) 105(2), 65 and Proc. Soc. Exp. Biol. & Med (1997) 215(1), 1)*.

There is evidence that CRF antagonist compounds and compositions, which can attenuate the physiological responses to stress-related phenomena, have potential therapeutic utility for the treatment of a wide range of stress-related disorders, such as depression, anxiety related diseases, post traumatic stress disorder, obsessive compulsive disorder, headache, eating and feeding disorders, anorexia nervosa, gastrointestinal diseases, irritable bowel syndrome, inflammatory diseases, immune suppression, HIV infections, Alzheimer's disease, hemorrhagic stress, drug and alcohol withdrawal symptoms, drug addiction and fertility problems.
It is known from patent application WO 98/54221 that a number of relatively large peptides have strong CRF antagonist activity (Astressin and analogs). These known peptides have a substantial number of amino acids of around 30, obtained by shortening CRF by 8-10 residues at the N-terminus.

Further shortening at the N-terminus results in a sharp decline of the antagonist activity. For example the peptide derived from Astressin by N-terminal shortening to 25-amino acids ("Astressin 16-41") has a reduced CRF₁ receptor affinity and an antagonistic activity which is at least a factor 1000 less in comparison with the known peptides of about 30 amino acids, suggesting that further shortening will lead to a further reduction of activity.

It has now surprisingly been found that further shortening at the N-terminus to much smaller peptides of less than 17 amino acids results in peptides which have interesting affinity in the CRF₁ receptor binding assay, although not as active as the large peptides of about 30 amino acids. Functional testing reveals that these smaller peptides have a rather potent antagonist activity on interaction with the CRF₁ receptor.

It is clear that these small peptides, consisting of about half the size of the very active known peptides have a number of advantages in comparison with the larger peptides.
The synthesis of such 12 to 16 amino acid containing peptides is faster and easier than the preparation of larger peptides. The reduced requirements for chemical building blocks, reagents and solvents results in a lower cost price. Furthermore the reduced number of scissile amide bonds in such peptides results in better stability of the peptides. A final and crucial advantage of such shorter peptides is their better suitability for deriving pharmacophoric information on the interaction with CRF receptors. This makes them very interesting as leads, for developing peptide ligands of still further reduced size or as leads for developing non-peptidic Low Molecular Weight ligands.

The invention relates to novel CRF antagonist peptides consisting of 12 to 16 amino acids which bind to CRF receptors having the formula 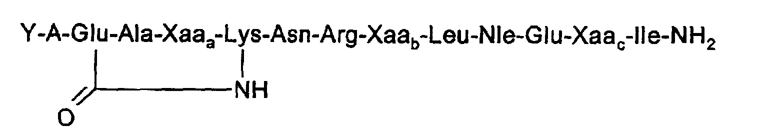 wherein Y is a hydrogen atom or an acyl group having up to 5 carbon atoms;
A is absent or represents
Gln-Leu-Ala-Gln,
Leu-Ala-Gln,
Ala-Ala-Gln
Ala-Ala-Ala or
Leu-Ala-Ala;
Xaaₐ is His or Ala; Xaa_{b} is Lys or Ala; Xaa_{c} is Ile or Ala (SEQ ID NO: 1-6);
and salts thereof, with the proviso that Xaa_{c} is not Ala when A is absent and that Xaa_{b} is not Ala when A represents Leu-Ala-Gln.

Preferably Y is acetyl and A is selected from the group having a length of 3 amino acids.

Pharmacologically acceptable acids with which the peptides of the invention can form suitable acid addition salts are for example hydrochloric acid, sulphuric acid, phosphoric acid, nitric acid, and organic acids such as citric acid, fumaric acid, maleic acid, tartaric acid, acetic acid, benzoic acid, p-toluene sulphonic acid, methanesulphonic acid and naphthalene sulphonic acid.

The peptides and their acid addition salts can be brought into pharmaceutical compositions suitable for administration by means of suitable processes using auxiliary substances such as liquid and solid carrier materials.

As mentioned the peptides described above and salts thereof have interesting CRF antagonistic activity. The peptides are especially useful as CRF₁ antagonists.

### In vitro receptor binding assay

The affinity of the compounds of the invention for the CRF receptor was determined by binding studies using membrane preparations of CC7-cells containing the human CRF receptor and [¹²⁵I]-ovine CRF as the ligand.
Separation of bound and free ligand is performed by filtration over glassfiber-filters, essentially as described by H.Herdon et al, *Soc. Neurosci. Abstracts 21(2),1349,1995*.
Radioactivity on the filter is measured by liquid scintillation counting. Results are expressed as IC₅₀ values and transformed into inhibitory constants (Ki) and expressed as pKi values. The compounds of the invention have affinities towards the CRF₁ receptor in the range of pKi = 7-9.

### Assay for functional CRF antagonism

The antagonistic activity of the compounds of the invention was determined by functional studies using LVIP cells (*Mol. and Cell Neuroscience 2:331, 1991)*. containing the rat CRF receptor. The origin of these cells is the mouse L-cell-line containing the cAMP responsive reporter gene construct coding for the enzyme β-galactosidase, which is subsequently transfected with the plasmid containing the genes coding for rat CRF.
Formation of cAMP is stimulated with rat CRF (10⁻⁸M) for 3 hours. The increase of cAMP results in an increase in the production of β-galactosidase by stimulation of the reporter gene. The yellow product formed after administration of the substrate O-nitrophenyl-β-D-Galactopyranoside is measured spectrophotometrically (405 nm). Antagonistic activity can be obtained after a 30 minutes pre-incubation with putative antagonists and subsequent incubation with the (reference) agonist CRF for 3 hours and is expressed as pA₂ values. The compounds of the invention have antagonistic potencies on Interaction with the CRF₁ receptor in the range of pA₂ = 6-8.

The invention is now further illustrated by means of the following Examples.

### Example I. Synthesis in general

### Instruments and methods.

Peptides are synthesized on an Applied Biosystems 433A Peptide Synthesizer (Foster City, California, USA). Analytical and preparative HPLC runs are performed on a Gilson HPLC workstation (Middleton, Wisconsin, USA), consisting of two Gilson 306 Pumps, a Gilson 811 C Dynamic Mixer, a Gilson 806 Manometric Module, a Gilson 119 UV/VIS detector, a Gilson 402 Syringe Pump and a Gilson 233XL On-line Column Switching Autoinjector (Fraction collector). Analytical HPLC runs are performed on Adsorbosphere XL C8, 5 µm particle size, 90 A pore size (I: 250 mm, i.d.: 4.6 mm) and Adsorbosphere XL C18, 5 µm particle size, 300 A pore size (I: 250 mm, i.d.: 4.6 mm). Preparative HPLC is performed on Adsorbosphere XL C18, 10 µm particle size, 300 A pore size (I: 250 mm, i.d.: 22 mm), Adsorbosphere column packing is a trademark of Alltech Corp. (Deerfield, Illinois, USA). The HPLC-runs are controlled by Gilson UniPoint™ System Software version 1.65 formatted for Windows95 on an IBM-compatible PC. Positive-ion Fast Atom Bombardment mass spectra (FAB-MS) are acquired using a Jeol JMS SX/SX 102 A four-sector mass spectrometer (Tokyo, Japan), operated at 10 kV accelerating voltage and producing a beam of 6 keV Xe atoms. Full mass spectra are recorded over about 15 s for the m/z 10 - m/z 3000 mass range by averaging 5 -10 scans. The data are acquired and processed with an HP 9000 Data System using Jeol Complement Software. The samples are dissolved in water and approximately 1 µl of the solution, containing about 0.1 µg of the compound, is mixed with a microdroplet of glycerol on the FAB probe. Compounds with a mass higher than m/z 3000 are measured by Electrospray Ionization Mass Spectrometry (ESI-MS) on a Micromass Platform II (Micromass UK Ltd., Altrincham, United Kingdom) single quadrupole bench-top mass spectrometer operating in a positive ionization mode. For the MS full scan spectra, data are acquired in continuum mode over the range m/z 600 to 3000 in 2 seconds at unit mass resolution. A cone voltage of 35 V is set, which produces predominantly (M + H)⁺ ions with little evidence of fragmentation for this type of peptides. Instrumental control, data acquisition and data processing are carried out using the MassLynx software package (version 2.3). The observed average m/z is compared with the calculated (M + H)⁺ average mass.

### Chemicals and Reagents

The peptides are synthesized on TentaGel® S RAM resin, 0.24 mmol/g (Rapp Polymere, Tübingen, Germany), functionalized with a 4-[(2',4'-dimethoxyphenyl)amino-methyl]phenoxyacetamido moiety (Rink amide linker) to obtain C- terminal amides (after TFA cleavage). N Fmoc-protected amino acids and HOBt are purchased from Advanced ChemTech Europe (Machelen, Belgium). The side chain protecting groups are chosen as: Arg(Pbf), Asn(Trt), Gln(Trt), Glu(OtBu), His(Trt) and Lys(Boc). Fmoc-Glu(OAll)-OH, Fmoc-Lys(Aloc)-OH and Fmoc-D-Phe-OH and are obtained from Neosystem Laboratoire (Strasbourg, France). The coupling reagents, HBTU and BOP are purchased from Richelieu Biotechnologies Inc. (Montreal, Canada). Peptide grade NMP, DCM and TFA and HPLC- grade CH₃CN are from Biosolve B.V. (Valkenswaard, The Netherlands). Piperidine, DIPEA, NMM and tetrakistriphenylphosphine palladium(0) are obtained from Acros Organics (Beerse, Belgium). TIPS and EDT are supplied by Merck (Darmstadt, Germany).
All other chemicals are at least analytical reagent grade.

### Peptide Synthesis

The peptides can be synthesized automatically on TentaGel® S RAM resin functionalized as described above (1 gram of resin for a synthesis on 0.25 mmol scale) with the aid of the 433A Peptide Synthesizer described above, using the FastMoc protocol (see Fields, C.G. et al., Peptide Res. (1991), 4, 95-104). Fmoc deprotection is monitored by UV absorbance (λ = 301 nm) of the dibenzofulvene-piperidine adduct using an Applied Biosystems 759A Absorbance Detector. The syntheses are controlled by Applied TM Biosytems SynthAssist™ version 2.0 formatted for Macintosh. Coupling of N^{α}-Fmoc amino acids (1 mmol, 4 eq) are performed with HBTU/HOBt in the presence of DIPEA (2 mmol, 8 eq) in NMP for 45 mm. Fmoc removal is carried out with 20% piperidine in NMP for 10 min. Any remaining amino groups after incomplete coupling and the final amino functionality, respectively, are acetylated by acetic anhydride/DIPEA/HOBt in NMP. All resin washes are carried out with NMP. The allyl functionalities are removed by swirling the resin in CHCl₃/acetic acid/NMM 95:5:2.5 v/v/v in the presence of 3 eq. of tetrakistriphenylphosphine palladium(0) under an argon atmosphere for 16 hrs. The resin is extensively washed with 10 ml DCM (three times, 2 min), 10 ml NMP (three times, 2 min), 10 ml 0.5% DIPEA in NMP (three times, 5 min), 10 ml 0.02 M N-diethyldithiocarbamic acid sodium salt in NMP (three times, 15 min), 10 ml NMP (five times, 2 min) and 10 ml DCM (three times, 3 min) to remove residual Pd catalyst. The ring closure between the unprotected side chains of Glu⁵ and Lys⁸ (SEQ ID NO:1), Glu⁴ and Lys⁷ (SEQ ID NO: 2-5) or Glu¹ and Lys⁴ (SEQ ID NO:6) is mediated on resin with 3 eq. of BOP/HOBt in the presence of 9eq. of DIPEA in NMP for 16 hours. Both the allyl removal and the ring closure reaction are monitored by the Kaiser test for the presence of free amine functionalities. Generally, the allyl removal is complete after 16 hrs. (A more precise analysis can be obtained if a small quantity of resin is treated with TFA/H₂0/TIS/EDT 85:8.5:2:4.5 v/v/v/v to deprotect and detach the peptide from the resin. After three hours the resin is filtered off and the TFA solution is diluted with diethyl ether/hexane 1:1 v/v to precipitate the peptide. This peptide is analyzed by HPLC and ESI-MS for the occurrence of remaining allyl functionalities.) Generally the ring closure is complete after 16 hours. The ring closure is complete if the Kaiser test yields no blue-colored resin beads. The peptides are cleaved from the resin and deprotected by treatment with TFA/H₂O/TIS/EDT 85:8.5:2:4.5 v/v/v/v at room temperature for 3 hrs. The peptides are precipitated with diethyl ether/hexane 1:1 v/v at -20 °C. The precipitates are decanted and subsequently washed with cold diethyl ether (three times) and finally lyophilized from tert.BuOH/H₂O 1:1 v/v or CH₃CN/ H₂O 1:1 v/v.

Crude lyophilized peptides are desalted by size exclusion chromatography on Sephadex LH 20 (I: 135 cm, i.d.: 2 cm) (Pharmacia, Uppsala, Sweden) with CH₃CN/H₂O 1:1 v/v as eluent at a flow rate of 30 ml/hr. Fractions are analyzed by analytical HPLC on peptide content, pooled and lyophilized. The desalted peptides (30-100 mg) are dissolved in a minimum amount of 0.1% TFA in CH₃CN/H₂0 8:2 v/v and loaded onto the preparative HPLC column. The peptides are eluted with a flow rate of 11.5 ml/min using a linear gradient of buffer B (70% in 80 min) from 20% buffer A (buffer A: 0.1% TFA in H₂O, buffer B: 0.1% TFA in CH₃CN/H₂O 8:2 v/v).

### Peptide Characterization.

Peptide purity can be analyzed by analytical HPLC at a flow rate of 1 ml/min using a lineair gradient of buffer B (100% in 30 min) from 100% buffer A (buffer A: 0.1% TFA in H₂0; buffer B: 0.085% TFA in CH₃CN/H₂0 95:5 v/v) in case of the C8 column or a linear gradient of buffer B (70% in 40 min) from 80% buffer A (buffer A: 0. 1 % TFA in H₂0; buffer B: 0. 1 % TFA in CH₃CN/H₂0 8:2 v/v) in case of the C18 column. Purity is determined to be 95% or higher. The peptides are characterized by mass spectrometry. The mass of each analogue is measured and the observed monoisotopic (M + H)⁺ values are correlated with the calculated (M + H)⁺ values (the masses are calculated using MacBioSpec version 1.0.1)

### Example II. Synthesis of "N-acetyl Astressin (27-41)" (Compound with SEQ ID NO: 2 with Xaaₐ is His, Xaa_{b} is Lys and Xaa_{c} is Ile

The synthesis of "N-acetyl-astressin (27-41)" started with 1.0 g of functionalized and Fmoc protected TentaGel® S RAM resin (0.25 mmol). The resin was treated with 20% piperidine in NMP in order to remove the Fmoc group. An Fmoc deprotection cycle lasted 10 min. After Fmoc-removal was complete, the first amino acid residue (Fmoc-Ile-OH) was dissolved in NMP and 2.8 ml of a solution of 0.36 M HBTU/HOBt in NMP was added followed by 1 ml of a solution of 2 M DIPEA in NMP. The whole solution was transferred into the reaction vessel where it was intensively mixed with the resin. One activation (= coupling) cycle lasted 45 min. After this reaction time the coupling of the amino acid residue was complete. This deprotection/activation cycle was successively repeated with the following N^{α}-Fmoc protected amino acid residues: Fmoc-Ile-OH, Fmoc-Glu(O^{t}Bu)-OH, Fmoc-Nle-OH, Fmoc-Leu-OH, Fmoc-Lys(Boc)-OH, Fmoc-Arg(Pbf)-OH, Fmoc-Asn(Trt)-OH, Fmoc-Lys(Aloc)-OH, Fmoc-His(Trt)-OH, Fmoc-Ala-OH, Fmoc-Glu(OAll)-OH, Fmoc-Gln(Trt)-OH, Fmoc-Ala-OH and Fmoc-Leu-OH. After the final amino acid was coupled, the Fmoc-group was removed as described and the resulting free amino functionality was acetylated with Ac₂O/HOBt/DIPEA in NMP for 20 min. Finally, the resin was washed with NMP (once) and DCM (twice) to remove any residual reagents.
For the construction of the lactam bridge (formed between the side chains of glutamic acid on position 30 and lysine on position 33) the allyl ester and the allyloxycarbonyl functionality respectively, needed to be removed. For this purpose, 500 mg resin (with the fully protected peptide still attached to it) was swirled in 40 ml of oxygen-free CHCl₃/AcOH/NMM 92.5:5:2.5 v/v/v. Through this suspension argon was bubbled for 15 min after which 200 mg of Pd⁰(Ph₃P)₄ was added under strictly oxygen-free conditions. After 16 hrs of swirling, the resin was filtered over a glass filter and washed as described in Example l to remove residual Pd catalyst. These washing steps were performed manually by bubbling a low flow of nitrogen through the suspension. After these washing steps, the allyl removal was checked in a qualitative way by the Kaiser test on free amine functionalities. After complete allyl removal the peptide-resin was suspended in NMP (by bubbling nitrogen through the reaction vessel) and BOP/HOBt/DIPEA 3/3/9 (equivalents to a free amine functionality) were added. After 8 hrs the solvent was removed by filtration and the resin was resuspended in NMP followed by the repeated addition of BOP/HOBt/DIPEA. After 16 hrs, the resin was filtered off and thoroughly washed with successively 10 ml NMP (three times, 2 min), 10 ml DCM (three times, 2 min), 10 ml iProp-OH (three times, 2 min), 10 ml NMP (three times, 2 min) and 10 ml DCM (three times, 2 min). The completion of the ring closure reaction was checked by the Kaiser test. After this the resin was dried in high vacuum.

Finally, the peptide was deprotected and detached from the resin by treatment of TFA/H₂0/TIS/EDT 85:8.5:2:4.5 v/v/v/v. 400 mg of resin was suspended in 5 ml of the TFA solution and stirred for 3 hrs at room temperature. After this period the resin was filtered off and washed with 5 ml of TFA (twice 2.5 ml). The peptide was precipitated from the TFA solution by dilution in 100 ml diethyl ether/hexane 1:1 v/v. The white precipitate was centrifuged and decanted. The peptide was washed by resuspending in diethyl ether and centrifugation (five times). Finally, the crude peptide was air-dried and dissolved in 50 ml tert.-BuOH/H₂0 1:1 v/v and lyophilized. The yield of the crude peptide was 72%.
The crude peptide was purified by preparative HPLC. 50 mg of peptide was dissolved in 4 ml buffer A (0.1% TFA in H₂O) and loaded onto the column (Adsorbosphere XL C18) at a flow of 10 ml/min. The gradient used started at 20% B in A to 90% B in A in 80 min (buffer B: 0.1 % TFA in CH₃CN/H₂0 8:2 v/v). Purified peptide could be obtained with a recovery of 53% in 38% yield (20.8 mg) The purity and identity of the peptide were analyzed by analytical HPLC and ESI-MS and/or FAB-MS respectively.

### Abbreviations:

HOBT: N-hydroxybenzotriazole; HBTU: 2-(lH-benzotriazol-l-yl)-1,1,3,3-tetra-methyluronium tetrafluoroborate; BOP: benzotriazole- 1-yl-oxy-tris-(dimethylamino)-phosphonium. hexafluorophosphate; DIPEA: diisopropylethylamine; NMM: N-methylmorpholine; TIS: triisopropylsilane; EDT: 1,2-ethanedithiol; NMP: N-methylpyrrolidinone; DCM: dichloromethane; TFA: trifluoroacetic acid; HPLC: high, performance liquid chromatography; Fmoc: 9-fluorenylmethyloxycarbonyl; Pbf. 2,2,4,6,7-pentamethyl-dihydrobenzofuran-5-sulfonyl; Trt: trityl; tBu: tert.-butyl; Boc: tert.-butyloxycarbonyl; All: allyl; Aloc: allyloxycarbonyl.

## Claims

1. Peptides of the general formula 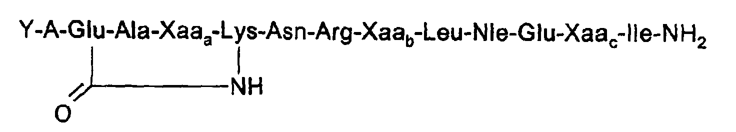 wherein Y is a hydrogen atom or an acyl group having up to 5 carbon atoms;
A is absent or represents
Gln-Leu-Ala-Gln,
Leu-Ala-Gln,
Ala-Ala-Gln
Ala-Ala-Ala or
Leu-Ala-Ala;
Xaaₐ is His or Ala; Xaa_{b} is Lys or Ala; Xaa_{c} is Ile or Ala (SEQ ID NO: 1-6);
and salts thereof, with the proviso that Xaa_{c} is not Ala when A is absent and that Xaa_{b} is not Ala when A represents Leu-Ala-Gln.

2. Peptides as claimed in claim 1, wherein Y is acetyl, A represents Leu-Ala-Gln, Ala-Ala-Gln, Ala-Ala-Ala or Leu-Ala-Ala and salts thereof.

3. Pharmaceutical composition which contains at least one peptide as claimed in claim 1 or 2 or a salt thereof as CRF antagonistic component.

4. Pharmaceutical composition according to claim 3, which contains at least of one of said peptides as CRF₁ antagonistic component.

5. Method of preparing a composition of claim 3 or 4, characterized in that an effective amount of a peptide of claim 1 or 2 or a salt thereof is brought into a form suitable for administration.

6. Method for attenuating the physiological response to stress-related phenomena, characterized in that a composition as claimed in claim 3 or 4 is used.
